# EUROPEAN PATENT APPLICATION

(11) **EP 2 777 496 A1**
(43) Date of publication of application: **17.09.2014**
(21) Application number: 14158685.9
(22) Date of filing: 10.03.2014
(51) Int. Cl.: A61B 5/08, G01N 33/00, G01N 33/497, A61B 5/00

(54) **Gas sensor device**

(30) Priority: 11.03.2013 GB 201304299
(71) Applicant: Bedfont Scientific Limited, Maidstone, Kent ME17 1JA (GB)
(72) Inventor: Smith, Trevor, Kent, ME17 1JA (GB); Smith, Jason, Kent, ME17 1JA (GB); Deamer-Smith, Scott, Kent, ME17 1JA (GB)
(74) Representative: HGF Limited

(57) **Abstract**

The present invention relates to a gas sensor device which is connectable to a portable electronic device, and to associated methods. The gas sensor device comprises a sensor adapted to detect a gas of interest in exhaled breath of a subject and provide an output signal which is dependent upon the concentration of gas detected, the gas sensor device comprising interface means adapted to communicate with the portable electronic device. It is preferred that the gas sensor device is connectable to the portable electronic device via an audio jack.

## Description

### Field of the Invention

The present invention relates to a breath carbon monoxide (CO) monitor for smoking cessation. It is an advantageous aspect of the present invention that the CO monitor can be connected to a portable electronic device, such as a mobile phone, for provision of on the spot motivation, education and validation of smoking status to a patient.

### Background to the Invention

Breath CO monitors measure the small amounts of CO in exhaled breath. The more an individual smokes, the higher their CO reading will be. CO monitors are currently used as a motivational tool by smoking cessation professionals. Monitoring an individual's breath CO levels indicates their level of nicotine dependency and can also act as a fool-proof marker of recent smoking activity.

Logging CO levels and showing how smoking cessation can reduce these to normal healthy levels can be used to motivate the individual during their endeavours to quit smoking. Each CO level band can be associated with a particular risk group and health-impairment factor.

However, it is a limitation of current devices that they are relatively bulky, are typically only available at a pharmacist or other health professional's premises. Thus, there is a need for devices which are more portable, user friendly, and simple to operate.

The present invention has adapted the power of 'smartphone' and tablet computer applications to provide an improved device. Using such a platform, the present invention provides an intelligent display and instant access to results recording and reporting platform that can be accessed by both the individual and the clinician as part of the on-going smoking cessation process.

### Brief Description of the Invention

According to a first aspect of the present invention there is provided a gas sensor device comprising a sensor adapted to detect a gas of interest in exhaled breath of a subject and provide an output signal which is dependent upon the concentration of gas detected, the gas sensor device comprising interface means adapted to communicate with a portable electronic device.

Suitably the interface means is adapted to be removably connectable to the portable electronic device. For example, the interface means comprises a connector which is removably connectable to a corresponding connector on the portable electronic device. For example, the interface means may comprise a plug which releasably connects with a socket on the portable electronic device.

Sensors for use in the gas sensor devices of the present invention as defined herein are sensors which both detect the presence of gas and provide a measureable value of the relative amount of gas detected, such as a voltage. The relative amount of gas as detectable by said sensors, is converted to an output signal readable by a portable electronic device by means as detailed hereinafter and thereafter converted to a relative measure of the concentration of gas, e.g. in parts per million (ppm), in the breath of the user via an application running on said portable electronic device.

Thus the sensor for use in the gas senor devices according to the first aspect of the present invention detects the presence of gas and provides a measureable output signal, the signal having a value which varies according to amount of gas detected.

Preferably the gas sensor is a CO sensor. Alternatively, other types of gas sensors and in particular gas sensors independently selected from: nitric oxide; methane; or hydrogen sensors, are also envisaged for utility in gas sensor devices as defined herein.

Preferably the gas sensor is an electrochemical sensor. Particularly preferred is an electrochemical CO sensor.

'Output signal' as defined herein is an electrical signal having a measureable value produced by the gas sensor. Suitably the output signal produced by the sensor is an output voltage proportional to the concentration of the gas of interest measured in exhaled breath.

Preferably the gas sensor device comprises analogue electronic circuitry associated with the sensor.

Preferably the gas sensor device comprises a signal converter to convert a primary output signal (e.g. voltage) from the gas sensor to a secondary signal suitable for transmission to, and in due course recognition by, a portable electronic device, via the interface means, e.g. by an audio jack connection. Suitably the secondary signal has a waveform with the frequency being proportional to the value of the primary output signal. 'Proportional' in this sense means that there is a definable relationship between the primary signal and the secondary signal, which can be used to convert one to the other. Typically the primary and secondary signals are directly proportional, but they could be indirectly proportional, geometrically proportional, or the like.

It is a significant advantage of the present invention that it allows information relating to the concentration of CO detected by the gas sensor to be transmitted through conventional audio circuitry and componentry used on conventional electronic devices. By converting the primary signal from the sensor into a secondary signal having a waveform with a frequency in the range typically used in audio (say 50 to 20,000 Hz, more typically from about 1 kHz to 10 kHz), this becomes possible.

Suitably the signal converter comprises a crystal controlled voltage to frequency converter integrated circuit, to generate a frequency that is proportional to the concentration of the gas of interest in the exhaled breath, as measured by the sensor.

This signal frequency is then transmitted down the interface means to the portable electronic device (e.g. smartphone), suitably via the microphone line, and converted to a concentration (e.g. a ppm reading) by a suitable application running on the portable electronic device. Suitably, each reading will be logged by the application along with the time and date it occurred. Preferably the application is adapted to provide a graphical display of all the results recorded when requested, e.g. as a graph of concentration against time.

The instant CO ppm reading and graphical display will assist a patient during a smoking cessation program because the CO levels can be measured at any time the patient choses without having to visit a clinic. The patient can also view his/her progress at any time by viewing the graphical display of past results.

Preferably CO levels measureable via the gas sensor devices according to the invention are in the region of from 0 to about 200 ppm, more preferably in the region of from 0 to about 100 ppm. Concentrations of CO in the exhaled air of non-smokers are typically up to a maximum of 10 ppm (even in polluted environments such as a city). Concentrations of CO in the exhaled air of smokers are typically up to above 10 ppm (for very light smokers), up to about 90 ppm (for very heavy smokers). It is therefore necessary that the device can detect concentrations between the extremes to cater for the majority of users.

Preferably the portable electronic device is an audio device comprising an audio connector. For example the audio device could be a portable media player. The portable electronic device is a suitably a device comprising a display and a programmable computing function. In particular the portable electronic device is preferably a smartphone, tablet computer or the like that runs a suitable operating system. Current operating systems which are conventionally used on smartphones and the like are iOS (from Apple, Inc.), Android (from Google, Inc.) or Windows Phone (from Microsoft Corporation).

Preferably the portable electronic device is programmed with a suitable application which can receive a signal from the gas sensor device and store or process it.

Preferably the interface means comprises, or is connectable to, a wire to connect the sensor device to the portable electronic device, and allow the transmission of a signal there between. Preferably the wire is adapted for the transmission of an analogue signal. Preferably the wire comprises an audio jack connector (also known as a phone plug, phone connector, phone jack, phone plug, jack plug, or phone plug) to allow connection of the sensor device to the portable electronic device. Said connection may be a direct connection via input of the connector into a corresponding socket of the portable electronic device, or via a line socket associated with a suitable input for the particular portable electronic device to be used.

In electronics, an 'an audio jack connector' is a common family of connectors typically used for analogue signals, most typically audio signals. It is cylindrical in shape, typically with three or four contacts, although versions with two contacts are also known. Three-contact versions are known as TRS connectors, where T stands for "tip", R stands for "ring" and S stands for "sleeve", these are also called stereo jacks, or headphone jacks. Similarly, four-contact versions are called TRRS connectors and are also known as 4 pole jacks, quad jacks or AV jacks.

In the present invention it is preferred that the audio jack connector is a 3- or 4-contact connector (i.e. a TRS or TRRS connector), most preferably a 4-contact connector. It is most preferred that the audio jack connector is a 3.5 mm audio jack connector (also known as a 3.5 mm jack plug, mini-stereo plug or headphone plug), i.e. the type which is conventionally used to connect headphones/microphones to personal audio equipment.

To ensure the subject can maintain their smoking cessation monitoring progress, preferably the gas sensor device an indicator to show of one or more of the whether the device is active (i.e. powered up) and the battery status (e.g. remaining battery life). The indicator can suitably be an LED display or other suitable indicator on said device.

Preferably the gas sensor device is adapted such that it can be switched on by a signal from the portable electronic device. Accordingly, a signal generated by the portable electronic device is utilised power up the circuitry and the sensor of the gas sensor device, i.e. to move the device from a 'stand-by' mode with negligible power consumption to an 'on' mode. The signal can suitably be an electrical signal having a suitable frequency, or any other suitable signal. For example, a 5 kHz signal generated constantly by the portable electronic device while the application is running can cause the sensor device to enter the on mode. When the signal stops, e.g. because the application is closed or the sensor device is disconnected, the sensor device returns to the stand-by mode.

In a preferred embodiment the gas sensor device comprises a housing which houses the gas sensor, the gas sensor being connected to a suitable power source (e.g. a lithium power cell) and suitable circuitry, the housing comprising a mouthpiece adapted for a user to blow through, the device further comprising interface means to allow electrical connection with a portable electronic device (preferably the audio circuitry of the portable electronic device).

In a preferred embodiment the mouthpiece is retractable, i.e. it is movable between an extended and a retracted position. In the retracted position the mouthpiece is preferably substantially entirely contained within the housing. In the extended position the mouthpiece extends from the device. Suitably the mouthpiece is slidably mounted on the housing. Suitably the device comprises an actuator to allow a user to move the mouthpiece from the retracted to the extended position. It is advantageous that the mouthpiece is retractable as it allows for easier storage in a more compact form, and also allows the mouthpiece to be kept clean.

Suitably the mouthpiece is D-shaped in cross section.

In a further aspect, the present invention provides a gas sensor device as described above connected to a portable electronic device.

Preferably the portable electronic device is a smart phone, tablet computer or the like.

Preferably the gas sensor device is connected to the portable electronic device via an audio connection on the device, in particular the headphone connection.

Preferably the portable electronic device contains a memory module having thereon a suitable application to operate the gas sensor device.

In further aspect there is provided a method of measuring a gas of interest in the exhaled air of a subject, the method comprising:
- providing a gas sensor device connected to a portable electronic device as described above;
- causing the subject to exhale through the gas sensor device;
- transmitting a signal from the gas sensor device to the portable electronic device through interface means (preferably via an audio jack connector);
- processing said signal to provide an output reading; and
- displaying the output reading on the portable electronic device.

Suitably the method comprises the step of converting a primary output signal from the gas sensor (e.g. a voltage), to a secondary signal having a waveform with a frequency proportional to the value of the primary signal. Suitably the frequency is in the range of from 50 to 20,000 Hz, more typically from 1 kHz to 10 kHz.

### Detailed Description of Embodiments of the Invention

The present invention will now be further described, by way of example only, with reference to the accompanying figures, in which:
- Figure 1 shows a representation of suitable circuitry to provide the signal conversion and recognition.
- Figure 2 shows a representation of a gas sensor device and a portable electronic device.
- Figures 3a to 3e show a representation of a gas sensor device in accordance with the present invention.

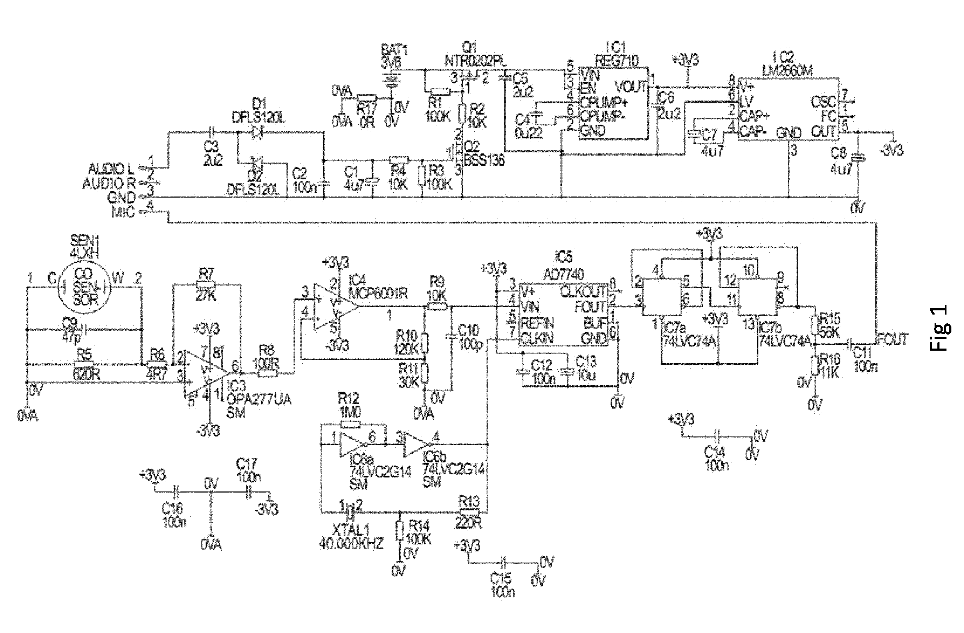

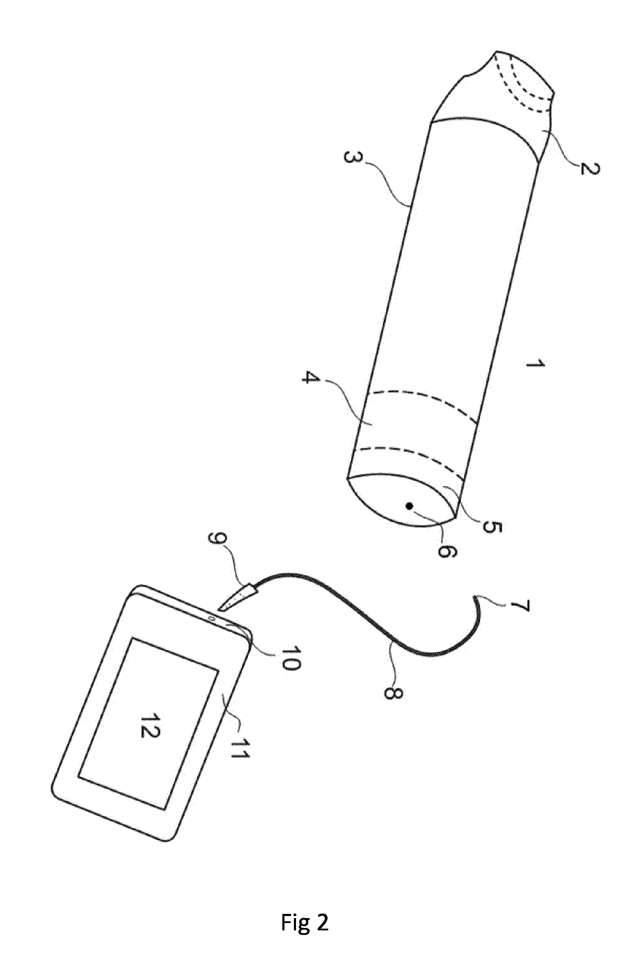

Figure 2 is a side perspective view representing a fully assembled example of a gas sensor device 1 according to a first aspect of the invention. As will be appreciated by the skilled man, the device 1 may be adapted to different scales in order to be suitable for use according to need. The illustrated example of a fully assembled device 1 in Figure 1 shows a housing 3 which encloses a gas sensor 4, the housing having located thereon at one end a mouthpiece 2, which surrounds one end of the housing 3. At the opposite end of the fully assembled device 1, from the mouthpiece is provided a socket 6 for connection to interface means 8 comprising a wire having a connector 7 for connection to the gas sensor device and having a TTRS jack connector 9 for connection to the portable electronic device 11 via a socket 10 thereon. Within the gas sensor device 1 are located a gas sensor 4 and signal conversion means 5.

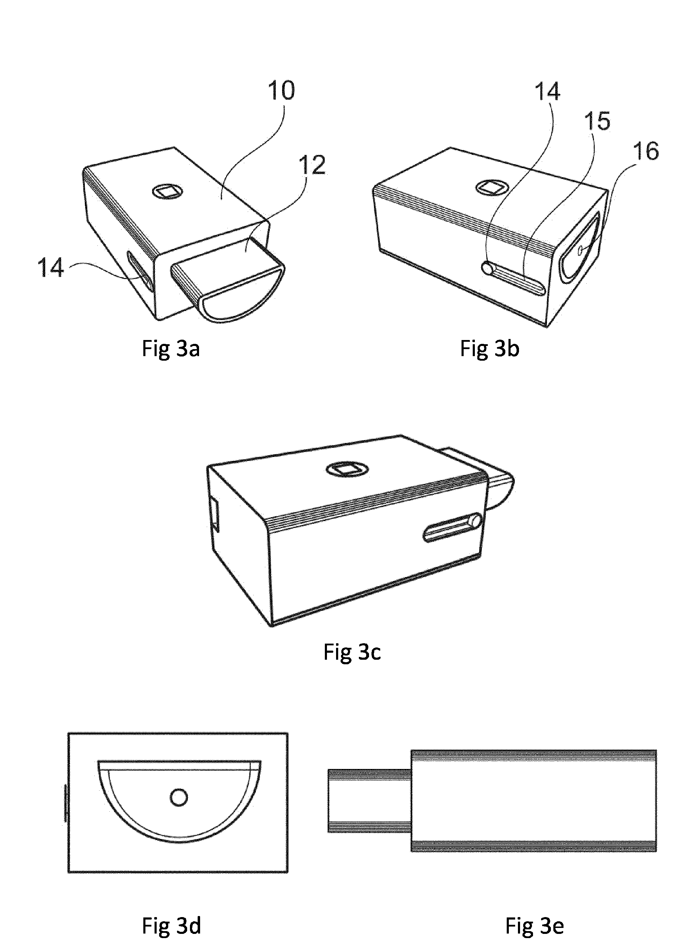

Figures 3a to 3e show a representation of a preferred embodiment of a gas sensor device according to the present invention. As can be seen, the device comprises a generally cuboidal housing 10. The housing contains the sensor and associated circuitry. The housing also comprises a retractable mouthpiece 12 at one end. The mouthpiece is slidably mounted on the housing to allow it to slide between an extended position (see Fig 3a) and a retracted position (see Fig 3b). An actuator 14 located in a slot 15 on the side of the device allows a user to move the mouthpiece between the retracted and extended positions. An aperture 16 can be seen which allows exhaled air from the user to pass into the housing to the sensor where the CO concentration is measured. The interface means is not shown in Fig 3a.

Figure 1 provides an illustration of circuitry suitable for recognition of a signal from a gas sensor device and conversion thereof to a suitable secondary signal for transmission to a portable electronic device (e.g. a smartphone) in accordance with the present invention. As will be appreciated by the skilled man, such circuitry may be applied to other portable electronic devices, or may be adapted to different scales for use therein without departing from the spirit and scope of the gas sensor devices of the present invention.

In Figure 1, transistors are indicated by Q, capacitors are indicated by C, resistors are indicated by R and voltage to frequency convertors are indicated by IC.

Transistor Q1 is switched on via transistor Q2. Transistor Q2 is switched on by a DC voltage of about 1.5 Volts derived from the voltage-doubling rectifier circuitry of C3, D1, D2, C2, C1 and R4. R3 serves to discharge the voltage when the signal from the Smartphone is switched off or removed. This will then turn off the battery supply.

The battery supply then feeds a Buck/Boost regulator to produce a regulated +3.3 V supply, formed by IC1 and associated components. A-3.3 V regulated supply is generated by the voltage inverter, IC2 and associated components, from the +3.3 V supply from the regulator.

The CO sensor output, SEN1, is conditioned and scaled by IC3, IC4 and associated components, to produce a voltage between 0 volts to about 2.5 volts proportional to 0 ppm to about 100 ppm CO. R9 and C10 form a simple low pass filter to remove noise and fluctuations in the signal.

The output from the low pass filter (R9 and R10) feeds the voltage input of the synchronous voltage to frequency converter IC5, and the reference clock input is generated from XTAL1, IC6a and IC6b and associated components. The frequency output generated by the synchronous voltage to frequency converter (IC5) is between about 4 KHz and about 36 KHz. This frequency range is beyond the input range of the microphone input of a Smartphone and is further divided by 4, using IC7a and IC7b, a dual D-Type flip-flop, producing a frequency range of about 1 KHz to about 9 KHz proportional to 0 ppm to about 100 ppm CO. Finally, to prepare a signal suitable for the smartphone microphone input, the frequency output signal is attenuated by R16 and R16 and then DC blocked by capacitor C11.

The electronic components of the circuitry detailed herein for the conversion of the gas sensor output signal into a suitable format for transmission to the selected portable electronic device are known components such as are available from a variety of commercial suppliers. It would be routine for the skilled person to select suitable components and configure appropriate circuitry to carry out the present invention in light of the teaching of the present invention. However, specific examples of suitable componentry are described below.

A buck-boost regulator, also known as a buck/boost regulator, or a buck/boost convertor is a regulator for switching voltage. Suitable buck-boost regulators for use herein are DC / DC convertors capable of supporting up to 4 A (Amp) of operating current and include :
Buck-boot regulators within the TPS6306 range, such as the TPS63060 available from Texas Instruments, see http:/www.ti.com/lsds/ti/power-management/buck-boost-converter-products.page?paramCriteria=no Voltage to frequency convertors (VCFs) are oscillators whose frequency are linearly proportional to control voltage. For accuracy synchronous charge balanced VCFs or so-called "sigma delta", Sigma-Delta VCD (SVCF) are utilised when output pulses are synchronized to a clock. Suitable synchronous voltage-to-frequency convertors (SVCDs) for use herein include the AD7741/AD7742 devices available from Analog Devices, Inc. http://www.analog.com/static/imported-files/data sheets/AD7741 7742.pdf

A D-type flip-flop device as used herein is a digital logic device that synchronizes changes in output state (1 or 0) according to a clocked input. D-type flip flops are negative-edge triggered devices having a 3-state output. Because they use sequential logic, flip-flops control and are controlled by other circuitry in a specific sequence that is determined by both a control clock and enable/disenable control signals. D flip-flops have one data input (D) and two outputs (Q and Q'). D flip-flops are pulsed with a clock. The D input is sampled during the clock pulse. If D = 1 when sampling occurs, the flip-flop is switched to the set state (Q = 1) unless it was already set. If D = 0, then the flip-flop switches to the clear or reset state (Q = 0). With D flip-flops, the no-change condition is attained only when there is no change to the D input.

Flip-flops vary in terms of supply voltage, operating current, propagation delay, power dissipation, low level output current (sink), high level output current (source), maximum clocking frequency, trigger type, and output characteristics. Supply voltages range from - 5 V to 5 V and include intermediate voltages such as -4.5 V, -3.3 V, -3 V, 1.2 V, 1.5 V, 1.8 V, 2.5 V, 3 V, 3.3 V, and 3.6 V. The operating current is the minimum current needed for active operation. The propagation delay is the time interval between the application of an input signal and the occurrence of the corresponding output. Power dissipation, the total power consumption of the device, is generally expressed in watts or milliwatts. The low-level output current (IOL) is the output current to which gates sink. The high-level output current (IOH) is the output current that gates source to a load. The maximum clocking frequency (fMAX) is the highest rate in hertz (Hz) at which flip-flops can be triggered reliably. Output enable (OE) inputs have an enable pin for the output.

Suitable commercially available D-type flip-flops for use in the present device include:
- the Flip Flops NB4L52MNGOS-ND available from Digi-Key Corporation http://www.globalspec.com/search/products?page=mi#comp=3537&vid=138662&sqi d=1179929 ; and
- the JM38510/07102BEA range of Dual J-K Negative-Edge-Triggered Flip-Flops With Preset And Clear 16-CDIP -55 to 125 available from Texas Instruments http://www.globalspec.com/search/products?page=mi#comp=3537&vid=385450&sqi d=1179929

It is a significant advantage that a 'hard-wired' connection is used between the sensor device and the portable electronic device, rather than a wireless connection. This is because it allows the energy consumption for the sensor device to be minimised. Typically it is conventional and desirable to connect accessories (other than audio headphones and microphones) to portable electronic devices via wireless connections, typically radio connections such as Bluetooth™. The advantages of such connections is that they are readily adapted to carry data and are ubiquitous in modern portable electronic devices; they would thus seem to be well suited to use in the present invention. However, the present inventors realised that such wireless communications systems consume considerably more power compared with hard-wired connections, and that the costs of integrating Bluetooth or the like into such a device was undesirable. An audio connection is not typically used for communicating data (other than audio signals or very rudimentary remote control functions) in portable electronic devices. However, the present inventors realised that by converting an electronic signal from a gas sensor (typically a voltage) to a suitable frequency, it would be possible to use an audio connection to link the gas sensor device to a portable electronic device. A significant advantage of using an audio connection in the present context is that audio connections are almost entirely standardised across portable electronic devices, being essentially solely implemented via a 3.5 mm jack connector. Another advantage is that the system required very little electrical power. Another advantage is that no 'pairing' or other such linking of the devices is required, which can be confusing for many users.

In use, the subject exhales into the mouthpiece of a gas sensor device, said mouthpiece being associated with a gas chamber, substantially as illustrated in Figure 3, which contains a commercially available electrochemical sensor (e.g. the 4LX CiTiceL CO Gas Sensor, part number: 2112B3008 available from City Technology Limited), powered by a 3.6V Lithium battery. The sensor detects the amount of CO in the exhaled breath of the subject and provides a voltage output signal which is proportional to the CO concentration (in ppm) in the exhaled breath of the subject. Associated analogue electronic circuitry within the gas sensor device, substantially as illustrated in Figure 1, converts this voltage output, via a crystal controlled voltage to frequency converted to a signal having a suitable frequency which is proportional to the CO concentration in the exhaled breath of the subject. Additional electronic circuitry within the gas sensor device and specifically a dual D-Type flip-flop voltage to frequency convertor, then divides the frequency output by about 4, producing a frequency range of about 1 KHz to about 9 KHz which is within detectable range of the microphone input of a typical smartphone. Additional circuitry tailors the signal further for the microphone input of the smartphone via attenuation and DC blocking of the frequency output signal (provided by R16 and R16, and Capacitor C11).

The signal is then transmitted from the gas sensor device to the smartphone via an interface means having a suitable audio jack connector compatible with the smartphone.

The frequency measured by the smartphone is then converted to a CO ppm reading by a specifically written application which logs the reading with the time and date and provides visual and recorded information of exhaled breath CO levels to assist the subject in their smoking cessation programme. With repeated usage of the exemplified gas sensor device, in conjunction with the smartphone and specifically written application running thereon, a historic log of CO ppm readings is built-up which can be accessed either in numerical or graphical format on the phone display.

The algorithm used for conversion of frequency (in Hz) to concentration (in ppm) is as follows: CO ppm = ((signal frequency) - 1000) / 80

Therefore, if a frequency of 4400Hz is generated by the iCO, then the CO ppm reading will be: (4400 - 1000) / 80 = 42.5 ppm of CO

The gas sensor device in this example is adapted such that a signal generated from the smartphone via its audio output will switch the device on and the device will automatically switch off when disconnected by the smartphone. Suitably the application causes the smartphone produces a 5 KHz signal to switch on the gas sensor device. This signal is turned off when the application is ended or the gas sensor device is disconnected.

The skilled man will recognise that whilst the examples illustrate a particular combination of commercially available mouthpiece, gas sensor, portable electronic device, and/or electronic components and/or connectors, the example device may comprise any alternative suitable commercially available mouthpiece, gas sensors, portable electronic devices and /or electronic components and/or connectors without departure from the spirit and scope of the present invention. In addition modified variants of commercially available items as defined herein before which perform the same, or substantially the same function are considered to be equally exchangeable for those detailed herein.

## Claims

1. A gas sensor device comprising a sensor adapted to detect a gas of interest in exhaled breath of a subject and provide an output signal which is dependent upon the concentration of gas detected, the gas sensor device comprising interface means adapted to communicate with a portable electronic device.

2. The gas sensor device according to any preceding claim wherein the interface means comprises a wire and an audio jack connector, preferably a 3- or 4-contact connector.

3. The gas sensor device according to any preceding claim wherein the gas sensor is a CO sensor.

4. The gas sensor device according to any preceding claim wherein the output signal produced by the sensor is an output voltage proportional to the concentration of the gas of interest measured in exhaled breath.

5. The gas sensor device according to any preceding claim which comprises a signal converter to convert a primary output signal from the gas sensor to a secondary signal suitable for transmission to, and in due course recognition by, a portable electronic device, via the interface means,
and preferably wherein the secondary signal has a waveform with the frequency being proportional to the value of the primary output signal, preferably wherein the secondary signal has a waveform with a frequency in the range 50 to 20,000 Hz.

6. The gas sensor device according to claim 5 which is adapted such that the secondary signal is transmitted via the interface means to a portable electronic device, suitably via the microphone line, for conversion to a concentration by a suitable application running on the portable electronic device.

7. The gas sensor device according to any preceding claim wherein the gas sensor is adapted to measure CO levels in the range the from 0 to 200 ppm.

8. The gas sensor device according to any preceding claim which is adapted such that it can be switched on by a signal from the portable electronic device.

9. The gas sensor device according to any preceding claim which comprises a housing which houses the gas sensor, the gas sensor being connected to a suitable power source and suitable circuitry, the housing comprising a mouthpiece adapted for a user to blow through, the device further comprising interface means to allow electrical connection with a portable electronic device.

10. The gas sensor device according to any preceding claim connected to a portable electronic device via the interface means, preferably via the headphone connection.

11. The gas sensor device according to claim 10 wherein the portable electronic device is a device comprising a display and a programmable computing function, and which is preferably a portable media player, smartphone or tablet computer that runs a suitable operating system.

12. The gas sensor device according to claim 10 or 11 wherein the portable electronic device is programmed with a suitable application which can receive a signal from the gas sensor device and store or process it.

13. The gas sensor device according to any one of claims 10 to 12 wherein the interface means comprises, or is connectable to, a wire to connect the sensor device to the portable electronic device, and allow the transmission of a signal there between, and wherein preferably the wire is adapted for the transmission of an analogue signal, and comprises an audio jack connector.

14. The gas sensor device according to any one of claims 10 to 13 configured such that each reading is logged by an application running on the portable electronic device along with the time and date it occurred, preferably wherein the application is adapted to provide a graphical display of all the results recorded when requested.

15. A method of measuring a gas of interest in the exhaled air of a subject, the method comprising:
- providing a gas sensor device connected to a portable electronic device according to any one of claims 10 to 14;
- causing the subject to exhale through the gas sensor device;
- transmitting a signal from the gas sensor device to the portable electronic device through interface means;
- optionally, converting a primary output signal from the gas sensor to a secondary signal having a waveform with a frequency proportional to the value of the primary signal, preferably wherein the frequency is in the range of from 50 to 20,000 Hz;
- processing said signal to provide an output reading; and
- displaying the output reading on the portable electronic device.
